# EUROPÄISCHE PATENTANMELDUNG

(11) **EP 2 638 933 A1**
(43) Veröffentlichungstag der Anmeldung: **18.09.2013**
(21) Anmeldenummer: 12159563.1
(22) Anmeldetag: 15.03.2012
(51) Int. Cl.: A61Q 19/00, A61K 8/97, A61Q 19/08, A61P 17/16, A61K 36/73

(54) **Kosmetikprodukte für die Altershaut**

(71) Anmelder: Kneipp-Werke Kneipp-Mittel-Zentrale GmbH & Co. KG, 97082 Würzburg (DE)
(72) Erfinder: Wohlfart, Rainer, 97320 Sulzfeld a. Main (DE); Blaak, Jürgen, 97084 Würzburg (DE); Simon, Isabel, 97252 Frickenhausen a. Main (DE)
(74) Vertreter: Keller, Günter

(57) **Zusammenfassung**

Offenbart wird ein kosmetisches Produkt mit einem pH-Wert von 3,0 bis 5,2, das sich speziell für die Behandlung der Altershaut sowie bei dort auftretenden Irritationen eignet. Es handelt sich bei dem kosmetischen Produkt bevorzugt um eine barrierestabilisierende Lotion oder eine barrierestabilisierende Creme, die einen von Frauenmantel (Alchemilla) erhaltenen Trockenextrakt enthält.

## Beschreibung

Das Durchschnittsalter der Bevölkerung steigt in den Industrieländern permanent an. Insbesondere die Zahl der über 60-jährigen wird in den nächsten Jahrzehnten stark zunehmen. Für diese Bevölkerungskreise müssen Produkte bereitgestellt werden, die den speziellen Anforderungen gerecht werden. Gerade bei alten und insbesondere bei sehr alten Menschen ist häufig zu beobachten, dass die Haut geschädigt ist oder durch leichteste Beanspruchungen beschädigt wird. Bei der Altershaut ist der physiologische pH-Wert häufig erhöht (Choi et al. (2007): Stratum corneum acidification is impaired in moderately aged human and murin skin. J Invest Dermatol 127: 2847-2856).

Gegenstand der vorliegenden Erfindung sind Kosmetikprodukte mit einem niedrigen pH-Wert, die einen positiven Einfluss auf epidermale Strukturen der Altershaut haben. Diese Produkte schützen, stabilisieren und erhöhen die Funktion der epidermalen Permeabilitätsbarriere. Bevorzugt handelt es sich bei den Kosmetikprodukten um barrierestabilisierende Emulsionen.

Der saure Hautoberflächen pH-Wert ist seit langem bekannt und liegt neuesten Studien zufolge zwischen 4,5 und 5. Dieser saure pH-Wert der Hautoberfläche steigt von außen nach innen an und beträgt zwischen Stratum corneum (Hornzellschicht) und Stratum granulosum (Körnerzellschicht) etwa 7. Durch diesen pH-Gradienten werden unterschiedliche Enzyme mit unterschiedlichem pH-Optimum gezielt in ihrer Aktivität gesteuert.

Enzyme mit einem pH-Optimum zwischen 4 und 5, wie z.B. β-Glucocerebrosidase und saure Sphingomyelinase sind von entscheidender Bedeutung für die Formation der interzellulären Lipidmatrix der epidermalen Permeabilitätsbarriere, die im Stratum corneum (SC) lokalisiert ist.

Ferner sind an der Desquamation (Abschuppung) der Hornzellen Serinproteasen beteiligt, die ein pH-Optimum von 7 haben. Bei einem Hautoberflächen-pH von 4,5 bis 5 sind diese Enzyme in ihrer Aktivität gehemmt, so dass es nicht zu einer übermäßigen Desquamation und damit zu einem Ungleichgewicht zwischen Desquamation und Zellerneuerung kommt. Darüber hinaus wirkt der saure Hautoberflächen-pH antimikrobiell. Die Normalflora der Haut hat sich dem pH 5 angepasst. Im Gegensatz dazu ist das Wachstum pathogener Keime, wie z.B. Staphylococcus aureus, bei pH 5 gehemmt. Somit sorgt der Hautoberflächen-pH zwischen 4,5 und 5 für eine Stabilisierung der "nützlichen" Normalflora und für eine Abwehr pathogener Keime. Ferner ist nachgewiesen, dass eine Erhöhung des Hautoberflächen-pH die unerwünschte Dissoziation (Entfernung) der Normalflora begünstigt.

Zusammenfassend ist der Hautoberflächen-pH zwischen 4,5 und 5 von zentraler Bedeutung für die Funktion der epidermalen Permeabilitätsbarriere, denn er reguliert die Barriereintegrität (Widerstandskraft der Barriere), die Barrierekohäsion (Zusammenhalt der Barriere), die Barriereregeneration nach Schädigung, sowie die antimikrobielle Abwehr.
(1) Eine Erhöhung des Hautoberflächen-pH hemmt die β-Glucocerebrosidase und die saure Sphingomyelinase und damit die Formation der interzellulären Lipidmatrix, der epidermalen Permeabilitätsbarriere.
(2) Eine Erhöhung des Hautoberflächen-pH steigert die Aktivität der Serinproteasen, gefolgt von einer übermäßigen Desquamation und damit verminderten Barriereintergrität und -kohäsion.
(3) Die Erhöhung des Hautoberflächen-pH hemmt das Wachstum der Normalflora, begünstigt das Wachstum pathogener Keime und dadurch bedingt die Entstehung dermaler Infektionen.

Bei den entzündlichen Hauterkrankungen (z.B. Ekzemerkrankungen oder Akne) ist der saure Hautoberflächen-pH erhöht. Bei der Haut von Neugeborenen (< 6 Wochen) und der Altershaut (>70 Jahre) liegt der Hautoberflächen-pH bei etwa 6-7.

In den 60er Jahren kamen zunehmend Hautreinigungsprodukte mit definiertem pH-Wert auf den Markt. Hintergrund war die Erkenntnis, dass der Hautoberflächen-pH-Wert stark durch Externa beeinflusst werden kann. Es wurden "pH-hautneutrale" Produkte zur Hautreinigung entwickelt. Diese Produkte waren und sind für die normale Haut bestimmt und sollen den normalen Hautoberflächen-pH stabilisieren. Von einigen Herstellern werden diese Produkte auch für empfindliche oder trockene Haut empfohlen.

Ein weiteres Gebiet, auf dem Kosmetika mit definiertem pH-Wert vermarktet werden, ist der Bereich des "Chemical Peeling". Durch das "Chemical Peeling" wird eine Ablösung der Hornzellen erreicht (Keratolyse), wodurch eine epidermale und dermale Regeneration angeregt wird. Das "Peeling" selbst wird mit extrem sauren Lösungen (pH-Wert 1-2) in der Arztpraxis durchgeführt. Die Vor-, Zwischen- und Nachversorgung der Patienten erfolgt mit Kosmetikprodukten mit einem pH-Wert von 3 bis 4.

Die erfindungsgemäßen Kosmetikprodukte unterscheiden sich von beiden oben beschriebenen Ausführungsformen:
1. Die erfindungsgemäßen Kosmetikprodukte sind nicht primär für die Anwendung an normaler Haut bestimmt, sondern eine spezielle Entwicklung für spezielle Hautzustände und inflammatorische Hauterkrankungen, die insbesondere bei einer reduzierten epidermalen Barrierefunktion auftreten.
2. Die Erfindung ist vom "Chemical Peeling" abzugrenzen, denn es soll durch die Erfindung gerade keine Keratolyse erreicht werden, daher haben die Produkte einen pH-Wert von wenigstens 3,0, bevorzugt wenigstens 3,2 und ganz besonders bevorzugt von wenigstens 3,5.
3. Die erfindungsgemäßen Produkte dienen einer speziellen Hautpflege für Hautzustände, deren gemeinsames Merkmal ein erhöhter Hautoberflächen-pH ist. Darüber hinaus ist den oben beschriebenen Hautzuständen gemeinsam, dass eine verminderte antimikrobielle Barriere und somit eine gesteigerte Anfälligkeit für Infektionen vorliegt.

Durch die Applikation der erfindungsgemäßen Kosmetikprodukte, insbesondere Cremes, Lotionen, Waschlotionen mit einem niedrigen pH-Wert kann der erhöhte Hautoberflächen-pH bestimmter Hautzustände und Hauterkrankungen in den normalen (physiologischen) Bereich zwischen 4,5 und 5 verschoben, respektive normalisiert und stabilisiert werden.

Durch die Normalisierung des Hautoberflächen-pH kann insbesondere die Funktion der epidermalen Permeabilitätsbarriere (Barriereintegrität, -kohäsion und -regeneration, relative Hornschichtfeuchte) verbessert werden, und die Funktion der antimikrobiellen Barriere verbessert werden.

Erfindungsgemäß wurden allgemein anerkannte Messverfahren der dermatologischen Forschung eingesetzt. Es wurden der Hautoberflächen-pH (Skin pH-Meter® 905; Courage & Khazaka, Köln, Deutschland), die relative Hornschichtfeuchte (Corneometer® CM 825; Courage & Khazaka, Köln, Deutschland), sowie der transepidermale Wasserverlust (Tewameter® TM 300; Courage & Khazaka, Köln, Deutschland) nach entsprechenden Richtlinien ermittelt (Parra et al. (2003): EEMCO guidance for the in vivo assessment of skin surface pH. Skin Pharmacol Appl Skin Physiol 16: 188-202, Berardesca (1997): EEMCO guidance for the assessent of stratum corneum hydration: electrical methods. Skin Res Technol 3: 126-132, Rogiers (2001): EEMCO guidance for the assessment of transepidermal water loss in cosmetic sciences. Skin Pharmacol Appl Physiol 14: 117-128). Darüber hinaus wurde die Überprüfung der epidermalen Barriereintegrität in Anlehnung an bereits publizierte Studien durchgeführt (Gunathilake et al. (2009): pH-Regulated Mechanisms Account for Pigment-Type Differences in Epidermal Barrier Function. J Invest Dermatol 129(7): 1719-1729).

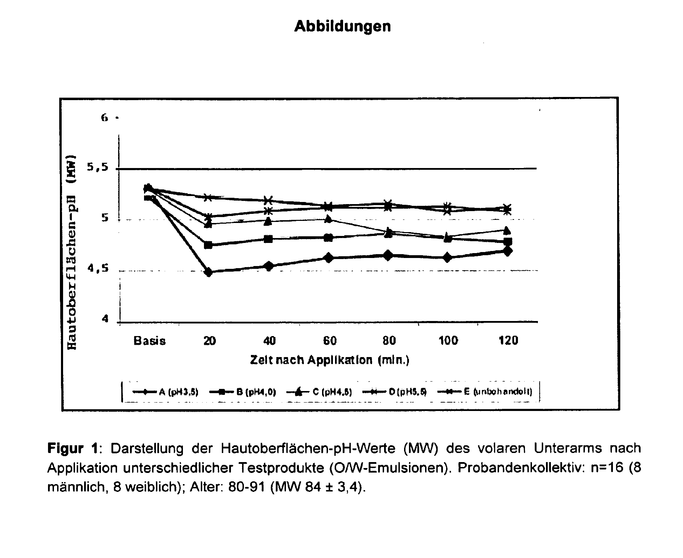

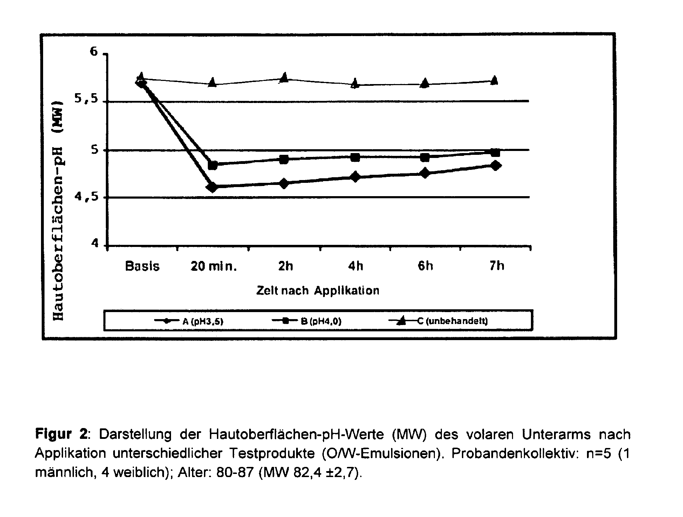

Die Überprüfung der kosmetischen Produkte wurde exemplarisch an der Haut von über 80-jährigen (Hochbetagten) durchgeführt. In Untersuchungen an der Haut von über 80-jährigen wurde gezeigt, dass mit einem erfindungsgemäßen kosmetischen Produkt der erhöhte Hautoberflächen-pH der Altershaut in den physiologischen Bereich zwischen pH 4,5 und 5 verschoben werden kann. Nach einmaligem Auftragen wurde der altersbedingt erhöhte Hautoberflächen-pH über 2 (Figur 1) bzw. über 7 Stunden (Figur 2) erniedrigt. Somit reicht wahrscheinlich die zweimalige tägliche Applikation des Pflegeproduktes aus, um eine kontinuierliche Normalisierung bzw. Stabilisierung des Hautoberflächen-pH zu erreichen. Dieser Effekt wurde durch eine handelsübliche Creme mit pH 5,5 nicht erreicht (Figur 1). Ferner wurde durch die 4-wöchige zweimalige tägliche Anwendung eines kosmetischen Produkts mit pH4 die Funktion der epidermalen Permeabilitätsbarriere verbessert. Es wurde untersucht, ob die 4-wöchige Anwendung des kosmetischen Produkts mit pH4 die Barriereintegrität der Altershaut verbessert.

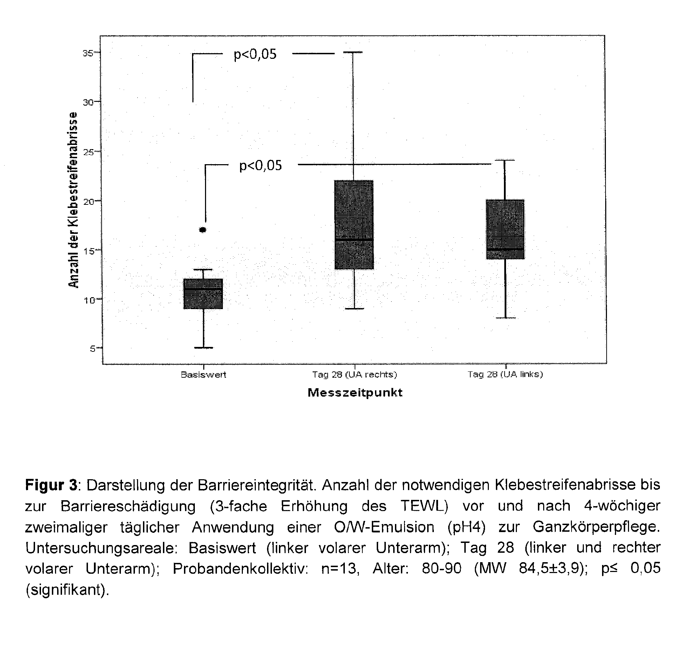

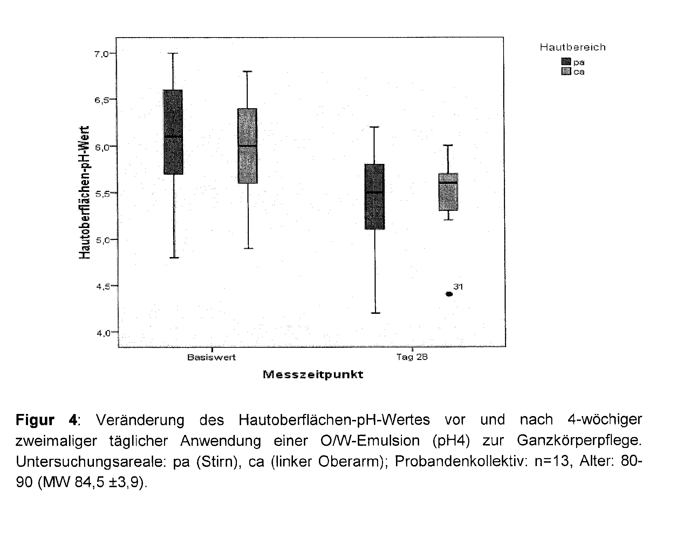

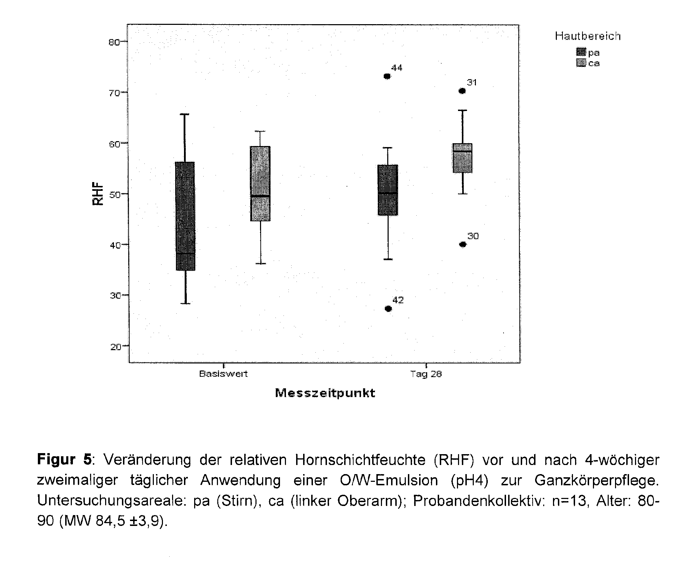

Die Barriereintegrität wurde überprüft, indem am Unterarm mit einem Klebestreifen (3M Blenderm surgical tape; 3M Deutschland; Neuss; Deutschland) Abrisse vorgenommen wurden. Das Abrissprocedere erfolgte in Anlehnung an Dickel et al. (2004): The "strip" patch test: results of a multicentre study towards a standardization. Arch Dermatol Res 296(5): 212-219, bis der transepidermale Wasserverlust (TEWL) um das 3-fache erhöht war. Die 3-fache Erhöhung des TEWL gilt als Indiz für eine epidermale Barriereschädigung (Gunathilake et al. (2009): pH-Regulated Mechanisms Account for Pigment-Type Differences in Epidermal Barrier Function. J Invest Dermatol 129(7): 1719-1729). Je weniger Abrisse erfolgen müssen um die Barriere zu schädigen (3-fache TEWL-Erhöhung), desto schlechter ist die Barriereintegrität. Vor der 4-wöchigen Therapie (s.o.) wurden durchschnittlich (Medianwert) 11 Klebestreifenabrisse benötigt um die Barriere zu schädigen, nach der Therapie waren 16 (rechter Unterarm) bzw. 15 (linker Unterarm) Klebestreifenabrisse notwendig (Figur 3). Das bedeutet, dass durch die 4-wöchige Anwendung eines kosmetischen Produkts mit pH4 bei über 80-jährigen (n=13) die Barriereintegrität signifikant verbessert wurde. Die 4-wöchige Anwendung des kosmetischen Produkts mit pH4 führte ferner zu einer signifikanten Erniedrigung des Hautoberflächen-pH und zu einer signifikanten Erhöhung der relativen Hornschichtfeuchte (Figuren 4-6).

Durch die erfindungsgemäßen Produkte wird die Beeinflussung und Stabilisierung des Hautoberflächen-pH von 4,5-5,0 durch die externe Zuführung von H⁺-Ionen erreicht, die den Produkten zugegeben werden. In bevorzugter Weise wird die Absenkung der pH-Werte durch Zugabe von physiologisch unbedenklichen Säuren, wie Milchsäure, Zitronensäure, Apfelsäure, Weinsäure, Salzsäure oder Phosphorsäure oder deren Salze, wie beispielsweise Di-Natrium-hydrogenphosphat oder Natrium-dihydrogenphosphat bewirkt.

Bei den Versuchen zum Einfluss des pH-Wertes wurden kosmetische Produkte verwendet, die hinsichtlich sämtlicher Komponenten identisch waren und sich nur im pH-Wert unterschieden. Die Einstellung des pH-Wertes erfolgte mit Zitronensäure. Der pH-Wert wurde nach auf dem Gebiet üblichen Methoden bestimmt und die Stabilität des eingestellten pH-Wertes wurde überwacht.

Gegenstand der vorliegenden Erfindung sind kosmetische Produkte, bevorzugt barrierestabilisierende Emulsionen, die als Wirkstoff einen Extrakt aus Frauenmantel (Alchemilla vulgaris) mit standardisiertem Polyphenolgehalt, bevorzugt in einer Menge von 0,025 bis 3 Gew.-%, besonders bevorzugt von 0,05 bis 0,5 Gew.-% und ganz besonders bevorzugt von 0,075 bis 0,2 Gew.-% des Trockenextrakts bezogen auf das fertige Produkt enthalten.

Bei den erfindungsgemäßen kosmetischen Produkten handelt es sich in bevorzugter Ausführungsform um barrierestabilisierende Emulsionen, wobei es sich hierbei in bevorzugter Ausführungsform um Emulsionen, in Form von Lotionen oder Cremes handelt.

Im Rahmen der vorliegenden Erfindung wurden verschiedene, auf unterschiedliche Art und Weise hergestellte Extrakte der Pflanze Alchemilla vulgaris hinsichtlich Phototoxizität, Zytotoxizität und antioxidativer Kapazität getestet. Bei diesen Versuchen stellte sich heraus, dass in besonders bevorzugter Ausführungsform ein wässriger Trockenextrakt aus Alchemilla vulgaris eine ausgeprägte antioxidative Kapazität aufweist.

Als Maß für die antioxidative Kapazität des erfindungsgemäß bevorzugt eingesetzten Trockenextrakts wurde der sogenannte ORAC-Wert (Oxygen Radical Absorbance Capacity) untersucht. Hierbei stellte sich heraus, dass der ORAC-Wert des erfindungsgemäß eingesetzten Trockenextrakts bevorzugt wenigstens 100000, besonders bevorzugt wenigstens 200000 und ganz besonders bevorzugt wenigstens 250000 µmol Trolox Äquivalente (TE)/100 g Extrakt aufweist. Die erfindungsgemäß eingesetzten Extrakte weisen einen nach der Methode ORAC FL gemessenen Wert von wenigstens 250000 µmol TE / 100 g, bevorzugt von wenigstens 280000 µmol TE / 100 g auf. Die Ergebnisse sind angegeben als "Trolox Äquivalente" (TE). Bei der Testmethode wird der oxidative Abbau eines fluoreszierenden Moleküls (beispielsweise Fluorescein) nach Mischung mit einer freie Radikale erzeugenden Verbindung gemessen.

In bevorzugter Ausführungsform weisen die erfindungsgemäßen barrierestabilisierende Emulsionen die im Folgenden aufgeführten Komponenten in den jeweiligen Anteilen bezogen auf das fertige Produkt auf:
- Emulgatoren werden in einer Menge von 1,5 bis 8, bevorzugt 2,5 bis 7 und besonders bevorzugt 3,0 bis 6,0 Gew.-% eingesetzt. Bei den Emulgatoren handelt es sich teilweise auch um Mischungen der im Folgenden aufgeführten Komponenten: Polyoxyethylenfettalkoholether, Polyglykolfettsäureester, Polyoxyethylenester mit modifizierten Fettsäuren; Cholesterol und Fettsäureester, Glyceryldilaurat, Glykolstearat, Dinatriumlaurethsulfosuccinat, Natriumdioctylsulfosuccinat, Alkoholethersulfat, Natriumalkylarylsulfonat, Polyethylenglykolalkylamine, quaternäre Ammoniumsalze, Cetearylalkohol und Polysorbat 20, Glycerylstearat, Kaliumpalmitoyl; hydrolysiertes Weizenprotein, hydrogeniertes Lecithin, Glyceryloleate.
- Mischungen verschiedener ungesättigter oder mehrfach ungesättigter Fettsäuren werden in einer Menge von 1,0 bis 40, bevorzugt 10 bis 35 und besonders bevorzugt 15 bis 25 Gew.-% eingesetzt. Die Fettsäuren können in der Form von Fettsäuren, Fettsäuresalzen oder in Form der Ester mit mehrwertigen Alkoholen wie Glycerin eingesetzt werden.
- Als Verdicker werden 0,1 bis 2, bevorzugt 0,2 bis 1 Gew.-% und besonders bevorzugt 0,25 bis 0,45 Gew.-% der folgenden Komponenten entweder einzeln oder im Gemisch eingesetzt: mikrokristalline Zellulose, Algin, Xanthangummi oder kosmetisch akzeptable Acrylate, wie C₁₀-C₃₀-Alkylacrylat-Crosspolymere.

Als Feuchthaltemittel wird in bevorzugter Ausführungsform 1,0 bis 20, bevorzugt 3,0 bis 10 Gew.-% und besonders bevorzugt 5,0 bis 8,0 Gew.-% wässriges Glycerin (85 % Glycerin) in pharmazeutisch anwendbarer Qualität zugegeben.
- Wenn zusätzlich noch Gerbstoffe eingesetzt werden, werden diese in einer Menge von 0,01 bis 1, bevorzugt 0,05 bis 0,5 und besonders bevorzugt 0,1 bis 0,2 Gew.-% zugegeben. Bevorzugte Polyphenole, wie Gerbstoffe sind charakteristisch für den Frauenmantel (*Alchimella vulgaris*). Frauenmantel zeichnet sich aus durch einen hohen Gehalt an Polyphenolen aus der Gruppe der Ellagtannine und Gallotannine (insgesamt zwischen 6-8%) aus. Wenn man den Gehalt der einzelnen Komponenten in Gewichtsprozent bezogen auf Drogentrockenextrakt angibt, weist der FrauenmantelTrockenextrakt, der bevorzugt erfindungsgemäß eingesetzt wird, einen Gehalt von 6-8 Gew.-% an Gerbstoffen auf. Bestandteile der Gerbstoffe sind Gallotannine, die in einer Menge von 1,0 bis zu 3,0 Gew.-%, bezogen auf den Drogentrockenextrakt vorliegen, sowie höhere Mengen an Ellagtanninen. Bei den Ellagtanninen handelt es sich insbesondere um Pedunculagin, das in einer Menge von 0,8 bis 1,5 Gew.-%, bevorzugt 1,0 bis 1,2 Gew.-%, bezogen auf den Drogentrockenextrakt vorliegt, um Agrimoniin, das in einer Menge zwischen 1,0 und 4,0 Gew.-%, bevorzugt 2,3 bis 3,6 Gew.-%, bezogen auf den Drogentrockenextrakt vorliegt sowie Laevigatin, das in einer Menge von 0,8 bis 1,0 Gew.-%, bevorzugt 0,85 bis 0,95 Gew.-% vorliegt.

Eine besonders bevorzugte barrierestabilisierende Emulsion der vorliegenden Erfindung beinhaltet folgende Komponenten in den angegebenen Mengenbereichen:

| **Bestandteil** | **Anteil in Gew.-%** |
|---|---|
| Emulgator | 4 |
| Pflanzenöl | 20 |
| Alchemilla vulgaris Trockenextrakt | 1% |
| Zitronensäure | zur pH Einstellung |
| pH-Wert | 4,0 ± 0,2 |

Die Ergebnisse der Beispiele sind in den Figuren dargestellt.
Figur 1 gibt den Hautoberflächen-pH des volaren Unterarms nach Applikation verschiedener Testprodukte über einem Zeitraum von 2 Stunden wieder. Es handelte sich hierbei um Öl in Wasseremulsionen, die sich lediglich im pH-Wert unterschieden. Weitere Wirkstoffe wurden hier nicht zugesetzt.
Figur 2 stellt die Auswirkung einer Creme mit niedrigem pH über einige Zeit nach der Applikation (bis zu sieben Stunden) dar. Aus Figur 2 ist ersichtlich, dass durch Verabreichung eines erfindungsgemäßen kosmetischen Produkts eine Absenkung des Hautoberflächen-pH unter 5 für mehrere Stunden bewirkt werden kann.
Figur 3 zeigt, dass nach 4-wöchiger Behandlung mit einem Produkt mit einem pH-Wert von 4 die epidermale Barriereintegrität verbessert werden konnte. Der Basiswert stellt die Kontrolle, also unbehandelte Haut dar.
Figur 4 zeigt, dass nach 4-wöchiger Behandlungsdauer bei zweimal täglicher Applikation der Hautoberflächen-pH abgesenkt werden konnte. Gemessen wurde der pH-Wert an überwiegend lichtgealterter (Stirn, zentral) und vergleichsweise an überwiegend zeitgealterter (Oberarm, ventral) Haut.
Figur 5 zeigt den positiven Effekt einer Creme mit pH 4 bei zweimaliger täglicher Applikation über eine Dauer von vier Wochen im Hinblick auf die Erhöhung der relativen Hornschichtfeuchte. Auch hier wurde an zwei verschiedenen Körperstellen (Stirn, Oberarm) gemessen.

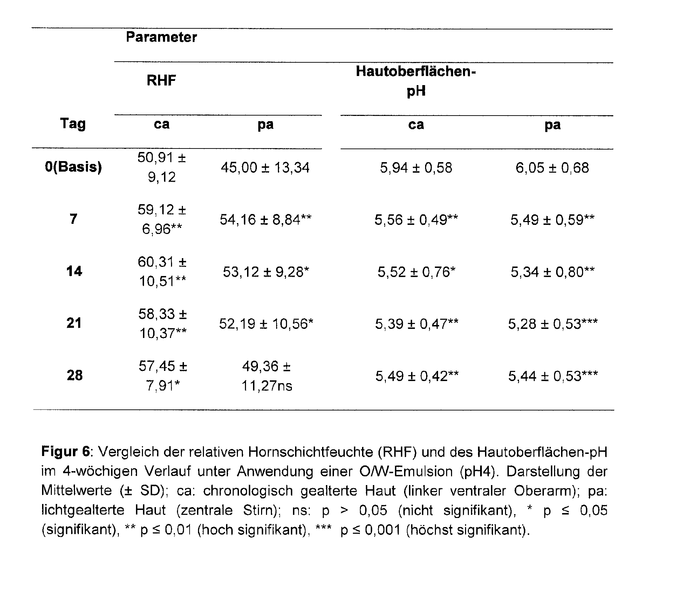

Bei Figur 6 sind die relativen Hornschichtfeuchtwerte und Hautoberflächen-pH unterschiedlicher Messzeitpunkte in tabellarischer Form dargestellt.

Im Rahmen der vorliegenden Erfindung konnte eine deutliche Verbesserung der Wirksamkeit durch den Zusatz eines Extraktes aus Frauenmantel erzielt werden.

Gegenstand der vorliegenden Erfindung sind daher kosmetische Produkte für die Altershaut mit einem pH-Wert von 3,0-5,2, die 0,1-5 Gew.-% eines Extraktes aus Frauenmantel bezogen auf das fertige Produkt enthalten.

Die erfindungsgemäßen Produkte weisen einen Anteil von 0,1-5 Gew.-%, bevorzugt 0,2-2,5 Gew.-% und besonders bevorzugt 0,2-1,0 Gew.-% eines Extraktes aus Frauenmantel auf, bezogen auf das fertige Produkt. Dies bedeutet, dass ein erfindungsgemäßes kosmetisches Produkt 0,1-5 g Extrakt aus Frauenmantel bezogen auf 100 g fertiges kosmetisches Produkt beinhaltet.

Frauenmantel gehört zu den Rosaceae und beinhaltet pharmakologisch aktive Inhaltsstoffe, insbesondere Gerbstoffe wie Gallotannine, Ellagitannine (Agrimoniin, Laevigatin, Pedunculagin), Flavonoide (Quercetinglykoside, Leukocyanidin), Phytosterine, sowie ätherische Öle. Diese Stoffe werden bei dem erfindungsgemäß eingesetzten Trockenextrakt angereichert.

Im Rahmen der vorliegenden Erfindung wurde festgestellt, dass ein Trockenextrakt aus Frauenmantelextrakt bevorzugt eingesetzt wird, weil dieser eine besonders gute Wirksamkeit hinsichtlich der antioxidativen Kapazität und der antizytotoxischen Wirksamkeit hat. Dies führt zu einer überraschend deutlichen Erhöhung der protektiven Wirksamkeit. Eine antioxidative Wirkung eines Extraktes von Alchemilla xantochlora wird von Filipek J et al., Pharmazie 47: 717-718 (1992): The effect of Alchimella xantochlora water extract on lipid peroxidation and superoxide anion scavenging activity. Pharmazie 47: 717-718) beschrieben.

Im Rahmen der vorliegenden Erfindung wurde überraschenderweise festgestellt, dass in kosmetischen Produkten, die auf einen pH-Wert zwischen 3,0 und 5,2, bevorzugt 3,2 bis 4,8 eingestellt wurden, durch den Zusatz eines wässrigen Extraktes von Frauenmantel eine wesentliche barrierestabilisierende Wirkung erzielt werden kann. Diese Ergebnisse treten zwar auch bei normaler und gesunder Haut auf, sind aber besonders ausgeprägt bei der Altershaut und ganz besonders ausgeprägt bei der geschädigten Altershaut. Bei Schädigungen der Altershaut treten vor allem Probleme auf, wie Xerosis cutis, Pruritus, Kontaktdermatitis, Ekzeme oder Pilzbefall, wie Candiasis.

In bevorzugter Ausführungsform wird der Trockenextrakt durch eine wässrige Extraktion der Droge erhalten. Hierbei werden die Pflanze bzw. die pharmazeutisch verwendbaren Pflanzenteile gesammelt und getrocknet. Die getrockneten und zerkleinerten Pflanzenteile werden dann mit Wasser extrahiert, wobei eine leichte Erwärmung bevorzugt auf eine Temperatur zwischen 30 und 50°C für die Effizienz der Extraktion hilfreich ist.

Die Wirkstoffe können aus der getrockneten oder auch frischen Pflanze nach verschiedenen an sich bekannten Verfahren hergestellt werden. Bevorzugt eingesetzt wird erfindungsgemäß ein wässriger Extrakt, bei dem die schonend getrockneten Pflanzenbestandteile, insbesondere die während der Blütezeit gesammelten oberirdischen Pflanzenteile mit Wasser extrahiert werden. Hierzu werden die Pflanzenteile schonend, gegebenenfalls unter Stickstoffatmosphäre zerkleinert und mit Wasser extrahiert. Die Pflanzenteile werden einige Zeit (1-12 Stunden) in Wasser belassen, wobei eine milde Erwärmung auf bevorzugt 30°C bis maximal 50°C bevorzugt wird. Anschließend wird das Wasser von den wasserlöslichen Bestandteilen getrennt und die wässrige Phase zur Trockene eingeengt. Wenn es sich in den Herstellungsprozess gut einfügt, kann auch wässriger Extrakt direkt in die kosmetischen Produkte eingearbeitet werden. Wichtig ist aber, dass die flüssigen Extrakte hinsichtlich der Trockenbestandteile standardisiert werden, so dass die erfindungsgemäße Konzentration an Frauenmantelextrakt eingehalten werden kann.

In bevorzugter Ausführungsform weisen die erfindungsgemäßen kosmetischen Produkte einen pH-Wert zwischen 3,2 und 4,8 auf, wobei ein pH-Wert zwischen 3,5 und 4,2 besonders bevorzugt ist.

In einer weiteren bevorzugten Ausführungsform weisen die erfindungsgemäßen kosmetischen Produkte weiterhin 0,5-2,5 Gew.-% Phytosterole, bezogen auf das Gesamtgewicht des fertigen kosmetischen Produkts, auf.

Bevorzugterweise wurden die Phytosterole erhalten aus einem Öl, ausgewählt aus der Gruppe bestehend aus Avocadoöl, Olivenöl und/oder Rapssamenöl. Die Phytosterole können in teilweise gereinigter Form eingearbeitet werden oder in Form der Öle, die reich an Phytosterolen sind, zugegeben werden.

Unter dem Begriff Phytosterole werden Steroide mit einer 3-β-Hydroxygruppe und einer Seitenkette mit 8-10 C Atomen am C17 Atom verstanden, die insbesondere aus Pflanzenöl isoliert wurden. In der Regel werden die Phytosterole als Mischungen eingesetzt, die einen überwiegenden Anteil an β-Sitosterol, einen wesentlichen Anteil an Campesterol und meist einen geringeren Anteil an Sigmasterol beinhalten.

Erfindungsgemäß ist es auch möglich den Phytosterolgehalt der eingesetzten Öle zu bestimmen und durch gezielte Auswahl der eingesetzten Öle den kosmetischen Produkten einen entsprechenden Anteil der Phytosterole zuzufügen. Eventuell noch fehlende Mengen können dadurch ergänzt werden, dass kommerziell erhältliche gereinigte Phytosterolkomplexe zugefügt werden, so dass die erfindungsgemäß bevorzugte Konzentration von 0,5-2,5 Gew.-%, noch bevorzugter 1,0-2,0 Gew.-% Phytosterol bezogen auf das fertige Produkt erreicht wird.

In einer weiteren bevorzugten Ausführungsform können die erfindungsgemäßen kosmetischen Produkte einen Gehalt an Quercetin von 10µg - 1000 µg bevorzugt 50 µg - 500 µg und besonders bevorzugt 100 µg - 250 µg pro 100g fertiges Produkt aufweisen.

Der erfindungsgemäß eingesetzte Frauenmantelextrakt beinhaltet üblicherweise 0,6-0,7 % Quercetin, bezogen auf den Trockenextrakt. Ergänzt kann dieser Gehalt werden, indem weitere Pflanzenextrakte zugegeben werden, die reich an Quercetin sind.

Quercetin ist ein zu den Flavonoiden gehörender Naturfarbstoff, der in vielen Pflanzen (Eiche, Äpfel, Weintrauben und andere) vorkommt. Bekannt ist, dass Quercetin eine antikancerogene Wirkung hat, die möglicherweise auf das antioxdative Potenzial zurückzuführen ist. Auch wenn die Wirkungsweise von Quercetin noch unbekannt ist, nimmt man an, dass es als Radikalfänger wirkt und reaktive Formen des Sauerstoffs (beispielsweise Superoxidanion) inaktiviert. Reaktive Formen des Sauerstoffs können auch in anderer Weise den Alterungsprozess der Haut beschleunigen. Daher verbessert die Zugabe von Quercetin die schützende und pflegende Wirkung der erfindungsgemäßen Produkte. Die Zugabe von Quercetin erfolgt entweder als Reinsubstanz oder bevorzugt als angereicherter Pflanzenextrakt. Bevorzugt eingesetzt werden Extrakte aus Eichenrinde, oder aus Blättern von Rosskastanie, Apfelbäumen oder Rosen.

In weiteren Ausführungsformen der vorliegenden Erfindung können auch Stoffe, wie zum Beispiel antiseptisch wirkende Stoffe, wie z.B. Chitosan eingearbeitet werden. Sinnvoll sind auch weitere antioxidativ wirkende Stoffe, wie z.B. die in Mangosteen vorkommenden Verbindungen. Derartige Stoffe werden erfindungsgemäß in einer Menge von 0,1 bis 5,0 Gew.-%, bevorzugt 0,2 bis 1,5 Gew.-% und besonders bevorzugt in einer Menge von 0,25 bis 0,5 Gew.-%, bezogen auf das fertige kosmetische Produkt, zugegeben.

Die kosmetischen Produkte liegen bevorzugt in Form einer barrierestabilisierenden Emulsion als Creme oder alternativ als Lotion vor. Der Ausdruck "barrierestabilisierende Emulsion" umfasst sowohl O/W-Lotionen und O/W-Cremen, wie auch W/O-Lotionen und W/O-Cremen.

Die erfindungsgemäßen kosmetischen Produkte können einerseits zur Verbesserung des Zustandes der Altershaut eingesetzt werden. Andererseits eignen sie sich aber ganz besonders zur Behandlung altersbedingter inflammatorischer Hautzustände.

Da es sich bei den erfindungsgemäßen kosmetischen Produkten in erster Linie um hautpflegende Produkte handelt, sind diese bevorzugterweise frei von Tensiden, die überwiegend zur Reinigung eingesetzt werden.

In einer bevorzugten Ausführungsform sind sämtliche Bestandteile der kosmetischen Produkte pflanzlichen Ursprungs. Solche Komponenten, die von Tieren, beispielsweise Talg, herstammen werden nicht verwendet. Dadurch kann eine potentielle Kontamination des Produktes mit potentiell pathogenen Agenzien wie Viren oder Prionen ausgeschlossen werden.

Die vorliegende Erfindung wird durch die nachfolgenden Beispiele weiter erläutert:

### Beispiel 1

Um den synergistischen Effekt von Emulsionen mit niedrigem pH-Wert und Frauenmantelextrakt zu belegen, wurde ein Versuch an gesunden jungen Probanden durchgeführt. Hierzu wurden zwei Emulsionen hergestellt. Die Ausgangsemulsion wies einen pH-Wert von 4,0 ± 0,1 auf und beinhaltete eine Emulsionsgrundlage, Wasser und als Vergleich 4 Gew.-% Vitamin E (α-Tocopherolum Acetat).

Die erfindungsgemäße Zusammensetzung beruhte auf derselben Salbengrundlage, wies einen pH-Wert von 4,0 ± 0,2 und als Wirkstoff 2 Gew.-% Vitamin E und 1 Gew.-% Frauenmanteltrockenextrakt auf.

Jeweils 10 Probanden applizierten eine der beiden Emulsionen 4 Wochen lang auf einen Unterarm. Anschließend wurden die Testareale UV exponiert (UVA: 4J/cm²). 48 Stunden später wurden die Testareale mittels Klebestreifenabrisse irritiert, bis der TEWL (Transepidermaler Wasserverlust) um das 3-fache angestiegen war. 24 und 48 Stunden postirritativ wurde der TEWL bestimmt (Maß der Barriereregeneration). Vergleichend ausgewertet wurden 4 Testareale:

| | Li Unterarm | | Re Unterarm | |
|---|---|---|---|---|
| | Areal 1 | Areal 2 | Areal 3 | Areal 4 |
| vorbehandelt | | | X | X |
| UVA exponiert | | X | X | |

Nachfolgend werden die Ergebnisse zusammengefasst.

Es wurde festgestellt, dass die Barriereregeneration der mit der erfindungsgemäßen Emulsion vorbehandelten Haut deutlich besser, d.h. schneller war als die mit der Vergleichsemulsion (nur Vitamin E als Wirkstoff) vorbehandelten Haut. Die erfindungsgemäße Emulsion bewirkt also 24 Stunden nach Exposition der Haut mit UVA-Licht eine bessere Barriereregeneration. Da die erfindungsgemäße Emulsion den hautphysiologischen pH-Wert gesunder junger Erwachsener nicht negativ beeinflusst, muss eine deutliche Verbesserung der Barrierefunktion gerade bei der älteren Haut angenommen werden. Bei kontinuierlicher Anwendung ist auch eine Zunahme der relativen Hornschichtfeuchte zu erwarten, so dass gerade bei der Altershaut eine deutliche Verbesserung der Barrierefunktion erwartet werden kann.

### Beispiel 2

Der in Beispiel 1 dargestellte Versuchsansatz wurde wiederholt. Es wurde eine barrierestabilisierende Emulsion hergestellt. Verglichen wurde einmal die barrierestabilisierende Emulsion, die einen pH-Wert von 4,0 ± 0,1 aufwies und keinen Wirkstoff enthielt, mit einer barrierestabilisierenden Emulsion, die im Unterschied zu der Vergleichszusammensetzung 1,0 Gew.-% eines durch wässrige Extraktion erhaltenen Trockenextrakts von Frauenmantel enthielt.

Die Versuche wurden durchgeführt wie in Beispiel 1 beschrieben. Überraschenderweise wurde festgestellt, dass die barrierestabilisierende Emulsion, die 1 Gew.-% durch wässrige Extraktion erhaltenen Trockenextrakt von Frauenmantel enthielt, eine viel bessere epidermale Barriereregeneration zeigte.

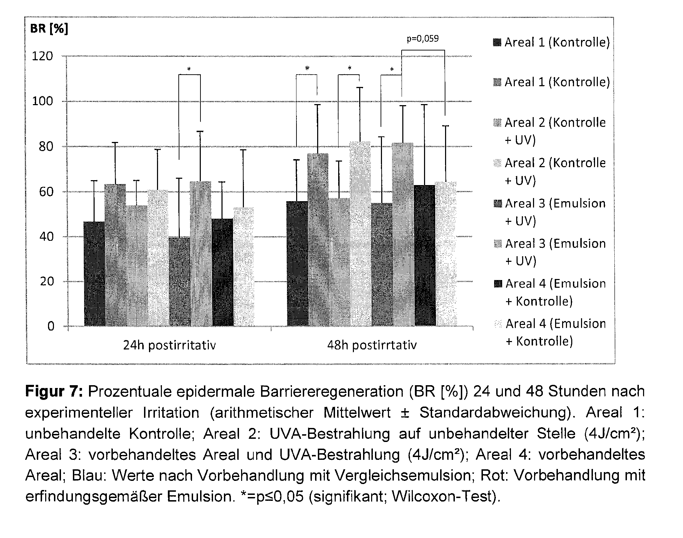

Die Ergebnisse dieses Versuchs sind in Figur 7 zusammengefasst. Die zugehörige Legende erläutert die Versuchsergebnisse.

## Patentansprüche

1. Kosmetisches Produkt mit einem pH-Wert von 3,0-5,2, **dadurch gekennzeichnet, dass** es 0,1-5 Gew.-% bezogen auf das Gewicht des Produktes eines Extraktes aus Frauenmantel enthält.

2. Kosmetisches Produkt nach Anspruch 1, **dadurch gekennzeichnet, dass** es einen pH-Wert zwischen 3,2 und 4,8 aufweist.

3. Kosmetisches Produkt nach Anspruch 1 oder 2, **dadurch gekennzeichnet, dass** es einen pH-Wert zwischen 3,5 und 4,2 aufweist.

4. Kosmetisches Produkt nach einem der vorhergehenden Ansprüche, **dadurch gekennzeichnet, dass** es sich bei dem Frauenmantelextrakt um einen Trockenextrakt handelt.

5. Kosmetisches Produkt nach Anspruch 4, **dadurch gekennzeichnet, dass** der Trockenextrakt durch eine wässrige Extraktion der Droge erhalten wurde.

6. Kosmetisches Produkt nach einem der vorhergehenden Ansprüche, **dadurch gekennzeichnet, dass** es 0,5-2,5 Gew.-% Phytosterole bezogen auf das Gewicht des fertigen Produktes aufweist.

7. Kosmetisches Produkt nach Anspruch 6, **dadurch gekennzeichnet, dass** die Phytosterole erhalten wurden aus einem Öl, ausgewählt aus Avocadoöl, Olivenöl und/oder Rapssamenöl.

8. Kosmetisches Produkt nach einem der vorhergehenden Ansprüche, **dadurch gekennzeichnet, dass** es einen Gehalt an Quercetin von 10 µg - 1000 µg pro 100 g fertiges Produkt aufweist.

9. Kosmetisches Produkt nach Anspruch 9, **dadurch gekennzeichnet, dass** es 2,5 bis 10 Gew.-% eines Emulgators bezogen auf das Gewicht des fertigen Produktes beinhaltet.

10. Kosmetisches Produkt nach einem der Ansprüche 1-9, **dadurch gekennzeichnet, dass** es sich um eine barrierestabilisierende Lotion handelt.

11. Kosmetisches Produkt nach einem der Ansprüche 1-9, **dadurch gekennzeichnet, dass** es sich um eine barrierestabilisierende Creme handelt.

12. Kosmetisches Produkt nach einem der Ansprüche 1-11 für die Behandlung der Altershaut.

13. Kosmetisches Produkt nach einem der vorhergehenden Ansprüche zur Behandlung altersbedingter inflammatorischer Hautzustände.

14. Verwendung eines kosmetischen Produkts nach einem der Ansprüche 1-13 zur Verbesserung der Funktion der epidermalen Permeabilitätsbarriere
